(19) Europäisches Patentamt / European Patent Office / Office européen des brevets

(11) **EP 2 418 004 A1**

(12) **EUROPEAN PATENT APPLICATION**

(43) Date of publication:
**15.02.2012 Bulletin 2012/07**

(51) Int Cl.:
***A61Q 19/08*** (2006.01) ***C11B 9/00*** (2006.01)

(21) Application number: **10172356.7**

(22) Date of filing: **10.08.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Hungkuang University
Shalu, Taichung County (TW)**

(72) Inventors:
• **Yih, Kuang-Hway
406, Taichun City (TW)**
• **Wang, Hsiao-Fen
510, Changhua County (TW)**

(74) Representative: **Becker Kurig Straus
Patentanwälte
Bavariastrasse 7
80336 München (DE)**

(54) **Essential oil composition with anti-free radical ability**

(57) Disclosed is an essential oil composition with anti-free radical ability, which includes dl-Citronellol, Eugenol, cis/trans-Citral, Eugenol Methyl Ether and Thymol. It is confirmed that the essential oil composition of the present invention has superior anti-free radical ability and anti-oxidative ability according to the indices including DPPH free radical scavenging ability, ABTS$^+$ free radical scavenging ability, metal ion chelating ability and reducing property, which are extensively used to determine the anti-oxidative ability of a substance.

**Description**

**BACKGROUND OF THE INVENTION**

**1. Field of the Invention**

The present invention relates generally to an essential oil composition and more particularly, to an essential oil composition having anti-free radical ability.

**2. Description of the Related Art**

Dr. Denham Harman, a professor of chemical in U.S., has shown in Drugs Aging in 1993 that excessive free radicals damage body cells and result in acceleration of aging process.Free radicals and the derivatives thereof constantly generate inside a living body because of the oxidative metabolism of a living body, the ion radiation existing in the environment and the pollutants such as air pollution and chemical substance. The skin of a human body is most easily affected by the ambient environment. For example, the mitochondria in the fibroblast in dermis of a human body will release superoxide anions ($O_2^-$) when they are stimulated by the ultraviolet light existing in the environment; consequently, those excess superoxide anions will transform to free radicals that seriously damage body cells. The antioxidation defense system of human body, which is used for maintaining the equilibrium between oxidation and antioxidation, is able to retard the generation of reactive oxygen species (ROS) and free radicals; however, a large number of free radicals that generate inside the human body because of exposure of sunshine for a long period of time will deteriorate the anti-oxidative ability of skin, resulting in acute or chronic damage of skin such as photoaging, epidermal wrinkle, immune imbalance of skin and skin cancer.

Although many products advertise that they can scavenge free radicals and have anti-oxidative ability through applying them onto the surface of skin to increase the anti-oxidative ability of human skin, the molecules of the anti-oxidative ingredients contained in most of the aforesaid products are larger or water-soluble such that it is hard for those molecules to be absorbed into human skin. Therefore, the actual anti-oxidative ability is limited.

On the other hand, various kinds of blend essential oils containing anti-oxidative ingredients are most popular to consumers because they have smaller molecules that can be easily absorbed into human skin through scenting incense, application, massage and immersion. As such, the anti-oxidative ingredients contained in a blend essential oil may be absorbed by skin cells and respiratory system and then penetrate into skin tissue to all cells. Nevertheless, no objective experimental data show that those blend essential oils indeed have free radical scavenging ability and anti-oxidative ability.

**SUMMARY OF THE INVENTION**

The main objective of the present invention is to provide an essential oil composition having anti-free radical ability. The essential oil composition of the present invention has superior anti-free radical ability and anti-oxidative ability than a commercial blend essential oil.

In order to achieve the aforesaid objective, the essential oil composition of the present invention includes dl-Citronellol, Eugenol, cis/trans-Citral, Eugenol Methyl Ether and Thymol.

Preferably, the essential oil composition may comprise 0.5-8 wt% of dl-Citronellol, 4.5-24.6 wt% of Eugenol, 3.75-20 wt% of cis/trans-Citral, 0.15-12.5 wt% of Eugenol Methyl Ether and 4.5-25.2 wt% of Thymol. More preferably, the dl-Citronellol may be contained in Geranium essential oil, the Eugenol may be contained in Cinnamon essential oil, the cis/trans-Citral may be contained in Lemongrass essential oil, the Eugenol Methyl Ether may be contained in Pimenta racemosa essential oil and the Thymol may be contained in Origanum vulgare essential oil.

Because all of the chemical compounds contained in the plant essential oils and used in the essential oil composition of the present invention have good DPPH free radical scavenging ability, ABTS$^+$ free radical scavenging ability, metal ion chelating ability and reducing property, the essential oil composition of the present invention exhibits superior anti-free radical ability and anti-oxidative ability.

**DETAILED DESCRIPTION OF THE INVENTION**

The present invention will become more fully understood from the detailed description given herein below.

According to the study of the present invention, an essential oil composition comprising dl-Citronellol, Eugenol, cis/trans-Citral, Eugenol Methyl Ether and Thymol provides a good anti-free radical ability.

An essential oil composition provided according to a preferred embodiment of the present invention comprises 0.5-8 wt% of dl-Citronellol, 4.5-24.6 wt% of Eugenol, 3.75-20 wt% of cis/trans-Citral, 0.15-12.5 wt% of Eugenol Methyl Ether and 4.5-25.2 wt% of Thymol.

Although phenols have good free radical scavenging ability, excessive phenols cause pungent odor. After lots of experiments, the above-mentioned ingredients and the content thereof were come out as an optimal solution in consideration of the balance between the free radical scavenging ability and the smell of the essential oil composition.

It will be appreciated that the contents of chemical compounds of the extracted essential oils may depend on the factors including, but not limited to, the source, cultivation, harvest time, extraction method and the extracted parts of plants for extraction. Essential oils containing an effective chemical compound in a specific content range can be used as the ingredients for the essential oil composition of the present invention.

More specifically speaking, for the dl-Citronellol, Geranium essential oil contains 5-40 wt% of dl-Citronellol can be used. For the Eugenol, Cinnamon essential oil contains 30-82 wt% of Eugenol can be used. For the cis/trans-Citral, Lemongrass essential oil contains 25-80% of cis/trans-Citral can be used. For the Eugenol Methyl Ether, Pimenta racemosa essential oil contains 0.1-5 wt% of Eugenol Methyl Ether can be used. For the Thymol, Origanum vulgare essential oil contains 30-84 wt% of Thymol can be used.

The essential oil compositions of the following examples are prepared respectively by blending the Geranium essential oil having 40 wt% of dl-Citronellol, the Cinnamon essential oil having 82 wt% of Eugenol, the Lemongrass essential oil having 80 wt% of cis/trans-Citral, the Pimenta racemosa essential oil having 5 wt% of Eugenol Methyl Ether and the Origanum vulgare essential oil having 84 wt% of Thymol in different ratios. There is no specific limitation to the method for blending these essential oils. Any method that can uniformly mix these essential oils can be used.

The present invention will be further described through the following examples which are used to more understand the present invention and are not used to limit the scope of the present invention. It will be apparent to one of ordinary skill in the art that many changes and modifications may be made thereto without departing from the spirit of the present invention.

**Example 1**

10 g of Geranium essential oil, 30 g of Cinnamon essential oil, 15 g of Lemongrass essential oil, 15 g of Pimenta racemosa essential oil and 30 g of Origanum vulgare essential oil were well blended to prepare 100 g of essential oil composition which comprises 4 wt% of dl-Citronellol, 24.6 wt% of Eugenol, 12 wt% of cis/trans-Citral, 0.75 wt% of Eugenol Methyl Ether and 25.2 wt% of Thymol.

**Example 2**

10 g of Geranium essential oil, 25 g of Cinnamon essential oil, 20 g of Lemongrass essential oil, 20 g of Pimenta racemosa essential oil and 25 g of Origanum vulgare essential oil were well blended to prepare 100 g of essential oil composition which comprises 4 wt% of dl-Citronellol, 20.5 wt% of Eugenol, 16 wt% of cis/trans-Citral, 1 wt% of Eugenol Methyl Ether and 21 wt% of Thymol.

**Example 3**

20 g of Geranium essential oil, 15 g of Cinnamon essential oil, 25 g of Lemongrass essential oil, 25 g of Pimenta racemosa essential oil and 15 g of Origanum vulgare essential oil were blended to prepare 100 g of essential oil composition which comprises 8 wt% of dl-Citronellol, 12.3 wt% of Eugenol, 20 wt% of cis/trans-Citral, 1.25 wt% of Eugenol Methyl Ether and 12.6 wt% of Thymol.

**Comparative Example 1**

The "Total Energy Essential Oil" of The Body Shop™, comprises Bergamot, Orange Sweet, Grapefruit, Rosewood, Palmarosa, Rosemary, Mint, Ginger, Lemon, Cedarwood and Patchouli.

**Comparative Example 2**

The "Aroma Oil No. 6" of DHC™, comprises Tangerine, Bergamot, Orange, Neroli Essence, Rosewood, Cedarwood and Sandalwood.

**Comparative Example 3**

The "Energy Stimulate Mix Organic Huiles Essentielles" of Le Bon moment™, comprises Juniper, Helichrysum, Rosewood, Rosemary and Mint.

**Comparative Example 4**

The "Chakra I" of Lorien Vana™, comprises Damask rose, Myrrh, Patchouli, Vetiver, Ginger and Rosewood.

**Comparative Example 5**

The "Chakra II" of Lorien Vana™, comprises Petitgrain, Geranium, Clary sage, Cypress, Rosewood and Grapefruit.

**Comparative Example 6**

The "Chakra VI" of Lorien Vana™, comprises Rose Geranium, Rosemary, Basil, Camphor, Highly Lavender and Geranium.

**Comparative Example 7**

The "Chakra VII" of Lorien Vana™, comprises Highly Lavender, Rosewood, Frankincense, Myrrh and Basil.

**Comparative Example 8**

The "Chakra VIII" of Lorien Vana™, comprises Highly Lavender, Grapefruit, Vetiver, Camphor and Bergamot.
The anti-free radical ability and anti-oxidative ability of the essential oil compositions of the examples and comparative examples are evaluated through the following measurements. The results are shown in Table 1 below.

**Measurement for DPPH free radical scavenging ability**

10 mg/mL solutions were prepared by using the compositions of examples 1-3 and comparative examples 1-8 respectively. Then, a $1.52 \times 10^{-4}$ M ethanol solution was prepared by dissolving 1, 1-Diphenyl-2-picrylhydrazyl (DPPH) in ethanol.
Thereafter, each sample mixture was prepared by vigorously shaking 500 $\mu$L of each of the aforesaid 10 mg/mL solutions and 2500 $\mu$L of the DPPH ethanol solution and placing in darkness at room temperature for 30 minutes.
In addition, the control mixture was prepared in the same manner as in the sample mixture, with the exception that the aforesaid 10 mg/mL solution was replaced by butyl hydroxy toluene (BHT).
The absorbance at 517 nm of the sample mixture and control mixture were measured respectively by UV-vis Spectrophotometer (HITACHI, UV-vis 2001). The scavenging effect of DPPH free radical was evaluated by using the following equation 1. The larger the value is, the stronger the DPPH free radical scavenging ability is.

[Equation 1]

$$\text{Scavenging ratio (\%)} = \left[ 1 - \left( \frac{\text{absorbance of sample mixture at 517 nm}}{\text{absorbance of control mixture at 517 nm}} \right) \right] \times 100$$

**Measurement for ABTS$^+$ free radical scavenging ability**

1 mg/mL solutions were prepared by using the compositions of examples 1-3 and comparative examples 1-8 respectively. Then, 250 $\mu$L of deionized water, 250 $\mu$L of 500 $\mu$M hydrogen peroxide solution, 250 $\mu$L of 1000 $\mu$M 2,2'-azino-bis(3-ethylbenzthiazoline-6-sulphonic acid) (ABTS) solution and 250 $\mu$L of 8.8 U/mL peroxidase were well mixed by Vortex Genie 2 Mixer (Scientific Industries). After about 10 minutes, when the stable ABTS$^+$ cation free radicals with aquamarine color generate, 250 $\mu$L of each the aforesaid 1 mg/mL solution was added therein to prepare mixtures. Thereafter, each the aforesaid mixture was well mixed by Vortex Genie 2 Mixer and incubated for 10 minutes to prepare each sample mixture.
In addition, butylated hydroxyl anisole (BHA) was used as a standard for the control mixture, and the aforesaid sample mixtures was replaced by 250$\mu$L of deionized water.
The absorbance at 410 nm of the sample mixture and control mixture were measured respectively by Enzyme-linked immunoassay (ELISA, Tecan Company, Sunrise-Basic-Tecan). The scavenging effect of ABTS$^+$ free radical was evaluated by using the following equation 2. The larger the value is, the stronger the ABTS⁺ free radical scavenging ability is.

[Equation 2]

$$\text{Scavenging ratio (\%)} = \left[ 1 - \left( \frac{\text{absorbance of sample mixture at 410 nm}}{\text{absorbance of control mixture at 410 nm}} \right) \right] \times 100$$

**Measurement for metal ion chelating ability**

1 mg/mL solutions were prepared by using the compositions of examples 1-3 and comparative examples 1-8 respectively. Then, each mixture containing each the aforesaid 1 mg/mL solution, 750 $\mu$L of 2 mM iron (II) chloride tetrahydrate solution and 250 $\mu$L of methanol was prepared by shaking for 30 seconds. Each the aforesaid mixture was added with 1500 $\mu$L of 5 mM ferrozine solution and placed in darkness for 30 minutes. After the reaction was completed, each mixture was subjected to centrifugation at 3000 rpm for 5 minutes and the supernatant liquid thus obtained was used as the sample mixture.
In addition, the control mixture was prepared in the same manner as in the sample mixture, with the exception that the aforesaid 1 mg/mL solution was replaced by ethylenediaminetetraacetic acid (EDTA) and the centrifugal process was not performed.
The absorbance at 562 nm of the sample mixture and control mixture were measured respectively by UV-vis Spectrophotometer (HITACHI, UV-vis 2001). The chelating effect of metal ion was evaluated by using the following equation 3. The larger the value is, the stronger the metal ion chelating ability is.

[Equation 3]

$$\text{Chelating ratio (\%)} = \left[ 1 - \left( \frac{\text{absorbance of sample mixture at 562 nm}}{\text{absorbance of control mixture at 562 nm}} \right) \right] \times 100$$

**Measurement for reducing property**

10 mg/mL solutions were prepared by using the compositions of examples 1-3 and comparative examples 1-8 respectively. Then, 2 mM phosphate buffer solution with pH 6.6 was prepared by mixing 0.2 M sodium dihydrogenphosphate solution and 0.2 M disodium hydrogenphosphate solution. Thereafter, 500 $\mu$L of each the aforesaid 10 mg/mL solution was mixed with 250 $\mu$L of the phosphate buffer solution and 250 $\mu$L of 1% Potassium Ferricyanide. The mixture was reacted at 50°C for 20 minutes in a water bath and then rapid cooled for 5 minutes. The obtained reactant was mixed with 250 $\mu$L of 10% trichloroacetic acid. 100 $\mu$L of the supernatant liquid thus obtained was mixed with 500 $\mu$L of deionized water and 750 $\mu$L of 0.1 % ferric chloride solution, and then the obtained mixture was placed in darkness for 30 minutes to prepare the sample mixture.
In addition, butylated hydroxyl anisole (BHA) was used as a standard for the control mixture, and the aforesaid sample mixture was replaced by 99.8% ethanol.
200 $\mu$L of the sample mixture and the control mixture were added into the wells of the test plate of Enzyme-linked immunoassay with 96-well respectively, and the absorbance at 700 nm of the sample mixture and control mixture were measured respectively by Enzyme-linked immunoassay (ELISA, Tecan Company, Sunrise-Basic-Tecan). The reducing property was evaluated by using the following equation 4. The larger the value is, the stronger the reducing ability is.

[Equation 4]

$$\text{Reducing ability (\%)} = \left[ 1 - \left( \frac{\text{absorbance of sample mixture at 700 nm}}{\text{absorbance of control mixture at 700 nm}} \right) \right] \times 100$$

Table 1

| | DPPH Scavenging ratio (%) | ABTS+ Scavenging ratio (%) | Chelating ratio (%) | Reducing (%) |
|---|---|---|---|---|
| Ex. 1 | 94.52±0.23 | 88.47±0.33 | 62.32±0.77 | 86.88±1.31 |
| Ex. 2 | 88.02±0.55 | 83.97±1.21 | 58.08±1.81 | 80.64±0.98 |
| Ex. 3 | 82.67±0.94 | 78.31±1.01 | 56.22±0.63 | 77.62±1.75 |
| Com. Ex.1 | 42.12±1.65 | 37.33±1.82 | 21.39±2.06 | 33.98±1.87 |
| Com. Ex. 2 | 28.24±0.98 | 27.88±0.45 | 18.65±0.77 | 25.85±1.74 |
| Com. Ex. 3 | 22.11±1.82 | 19.38±2.27 | 11.34±0.69 | 16.15±0.71 |
| Com. Ex. 4 | 38.32±0.83 | 36.18±1.28 | 28.7±0.22 | 18.28±0.06 |
| Com. Ex. 5 | 44.12±0.69 | 40.37±0.24 | 27.68±0.69 | 14.17±0.13 |
| Com. Ex. 6 | 48.35±0.63 | 44.97±0.68 | 55.31±0.52 | 20.21±0.92 |
| Com. Ex. 7 | 33.62±0.12 | 30.92±0.77 | 51.32±1.37 | 13.80±1.45 |
| Com. Ex. 8 | 28.12±0.31 | 26.41±0.32 | 19.55±0.61 | 16.61±0.44 |

As shown in Table 1, DPPH free radical scavenging ability, ABTS+ free radical scavenging ability, metal ion chelating ability and reducing property of examples 1-3 are much higher than those of comparative examples 1-8.

In addition, a human experiment was performed by the essential oil composition of example 3 through scenting incense, and the results show that the essential oil composition of the present invention is capable of scavenging the free radicals in human blood. The bloods of 15 adults before scenting incense and after scenting incense for 10 minutes were analyzed in the human experiment. First, 0.2 mL of blood was well mixed with 0.1 mL of 0.9% saline solution, and then the mixture was heated to 37 °C so as to simulate the body temperature. Thereafter, 1 mL of the reagent, which was prepared by dissolving 2.5 mg lucigenin in 0.9% saline, was added into the mixture. The amount of free radicals in the obtained mixture was measured by Chemiluminescence Spectrometer (Tohoku Electronic Co., Ltd). The results are shown in Table 2.

Table 2

| No. | Amount of free radicals before scenting incense | Amount of free radicals after scenting incense | Scavenging ratio (%) |
|---|---|---|---|
| 1 | 12136 | 5554 | 54.24% |
| 2 | 15038 | 3020 | 79.92% |
| 3 | 6283 | 5247 | 16.49% |
| 4 | 20511 | 3840 | 81.28% |
| 5 | 90235 | 31152 | 65.48% |
| 6 | 98326 | 2144 | 97.82% |
| 7 | 33897 | 14113 | 58.37% |
| 8 | 59844 | 5877 | 90.18% |
| 9 | 10673 | 3725 | 65.10% |
| 10 | 26632 | 8820 | 66.88% |

(continued)

| No. | Amount of free radicals before scenting incense | Amount of free radicals after scenting incense | Scavenging ratio (%) |
|---|---|---|---|
| 11 | 15351 | 7123 | 53.60% |
| 12 | 47392 | 15997 | 66.25% |
| 13 | 27340 | 2687 | 90.17% |
| 14 | 39810 | 2357 | 94.08% |
| 15 | 8991 | 4689 | 47.85% |
| Mean Value | 34164 | 7756 | 69% |

As shown in Table 2, the essential oil composition of example 3 is effective in scavenging the free radicals in human blood, and the scavenging effect is excellent based on the fact that the average free radical scavenging ratio is about 70%.

In conclusion, the essential oil compositions of examples 1-3 have superior DPPH free radical scavenging ability, ABTS+ free radical scavenging ability, metal ion chelating ability and reducing property. Besides, the free radical scavenging effect of the present invention is excellent in view of the aforesaid human experiment. Therefore, the present invention exhibits superior anti-free radical ability and anti-oxidative ability.


**Claims**

1.  An essential oil composition, **characterized in that** the essential oil composition comprises dl-Citronellol, Eugenol, cis/trans-Citral, Eugenol Methyl Ether and Thymol.

2.  The essential oil composition as claimed in claim 1, **characterized in that** the essential oil composition comprises 0.5-8 wt% of said dl-Citronellol, 4.5-24.6 wt% of said Eugenol, 3.75-20 wt% of said cis/trans-Citral, 0.15-12.5 wt% of said Eugenol Methyl Ether and 4.5-25.2 wt% of said Thymol.

3.  The essential oil composition as claimed in claim 1, **characterized in that** said dl-Citronellol is contained in Geranium essential oil.

4.  The essential oil composition as claimed in claim 1, **characterized in that** said Eugenol is contained in Cinnamon essential oil.

5.  The essential oil composition as claimed in claim 1, **characterized in that** said cis/trans-Citral is contained in Lemongrass essential oil.

6.  The essential oil composition as claimed in claim 1, **characterized in that** said Eugenol Methyl Ether is contained in Pimenta racemosa essential oil.

7.  The essential oil composition as claimed in claim 1, **characterized in that** said Thymol is contained in Origanum vulgare essential oil.

Europäisches
Patentamt

European
Patent Office

Office européen
des brevets

# EUROPEAN SEARCH REPORT

**Application Number**

EP 10 17 2356

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/153888 A1 (LEVERETT JESSE C ET AL) 13 July 2006 (2006-07-13) * paragraphs [0002], [0004], [0006]; examples 6,11; table 5 * ----- | 1-7 | INV. A61Q19/08 C11B9/00 |
| X | DE 103 05 965 A1 (BEIERSDORF AG) 26 August 2004 (2004-08-26) * paragraphs [0001], [0015] - [0022] * ----- | 1-7 | |
| A | WO 2007/125216 A1 (WESTRAND INTERNAT SARL) 8 November 2007 (2007-11-08) * example III * ----- | 1-7 | |

| TECHNICAL FIELDS SEARCHED (IPC) |
|---|
| A61Q C11B |

The present search report has been drawn up for all claims

| Place of search | Date of completion of the search | Examiner |
|---|---|---|
| Munich | 21 January 2011 | Hillebrecht, Dieter |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ..........................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 17 2356

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2011

| Patent document cited in search report | | Publication date | Patent family member(s) | | Publication date |
|---|---|---|---|---|---|
| US 2006153888 | A1 | 13-07-2006 | NONE | | |
| DE 10305965 | A1 | 26-08-2004 | CN KR | 1535676 A 20040073385 A | 13-10-2004 19-08-2004 |
| WO 2007125216 | A1 | 08-11-2007 | FR | 2900652 A1 | 09-11-2007 |

EPO FORM P0459

For more details about this annex : see Official Journal of the European Patent Office, No. 12/82